# EUROPEAN PATENT SPECIFICATION

(11) **EP 1 621 566 B1**
(45) Date of publication and mention of the grant of the patent: **04.07.2007**
(21) Application number: 05016109.0
(22) Date of filing: 25.07.2005
(51) Int. Cl.: C08G 59/24, C08G 73/06, C07D 265/16, H01L 21/56, H01L 23/29

(54) **Fluxing no-flow underfill composition containing benzoxazines**
Nichtfliessende Unterfüllungszusammensetzung mit Lötflusseigenschaften, welche Benzoxazine enthält
Composition de remplissage sous-jacent fluxante, sans ecoulement, contenant des benzoxazines

(30) Priority: 29.07.2004 US 902555
(43) Date of publication of application: 01.02.2006
(73) Proprietor: National Starch and Chemical Investment Holding Corporation, New Castle, Delaware 19720 (US)
(72) Inventor: Zhang, Ruzhi, Somerset New Jersey 08873 (US); Musa, Osama M., Hillsborough New Jersey 08844 (US); Bonneau, Mark, Brea California 92823 (US)
(74) Representative: Held, Stephan

(56) References cited:
- WO-A-01/34581
- WO-A-03/072638
- US-A1- 2004 087 681
- US-B1- 6 207 786

## Description

This invention relates to underfill encapsulant compositions containing benzoxazines, epoxies and anhydrides to protect and reinforce the interconnections between an integrated circuit (IC) chip and a substrate in a semiconductor package, or between a semiconductor component and a substrate in a microelectronic device.

Microelectronic devices contain integrated circuit components that are connected electrically to, and supported on, a carrier or a substrate, such as a leadframe or a printed wire board. There are a number of variations in the architecture of integrated circuit components, two of which are known throughout the industry as flip-chip and ball grid array. With these two, as with other integrated circuit components, electrical connections are made between electrical terminations on the integrated circuit component and corresponding electrical terminations on the substrate.

One method for making these connections uses polymeric or metallic solder material that is applied in bumps to the electrical terminals on either the component or the substrate. Solders are subject to oxidation and in consequence a fluxing agent is added to the component (silicon chip) or substrate. The terminals are aligned and contacted together and the resulting assembly is heated to reflow the metallic or polymeric material and solidify the connection.

A long-standing problem in this type of interconnect is the mismatch of the coefficients of thermal expansion (CTE) between the integrated circuit component, the interconnect material, and the substrate. To alleviate this mismatch and support the polymeric or metallic interconnects, encapsulant materials are filled in the interstices between the component and the substrate around the polymeric or metallic solder. These materials are known as underfills and their use enhances the fatigue life of the solder joints.

In a conventional underfill application known as capillary flow, the underfill dispensing and curing takes place after the reflow of the metallic or conductive polymeric solders and the formation of interconnection. In this procedure, before the terminals are aligned and contacted together, the fluxing agent or material is dispensed onto the terminals on the substrate. The terminals on the semiconductor chip and substrate are aligned, and the assembly is heated to reflow the solder joint. For metallic interconnects, such as eutectic or lead-free solder, this is at approximately 190° to 230°C.

At this point, a measured amount of underfill encapsulant material is dispensed along one or more peripheral sides of the electronic assembly and capillary action within the component-to-substrate gap draws the material inward. After the gap is filled, additional underfill encapsulant may be dispensed along the complete assembly periphery to help reduce stress concentrations and prolong the fatigue life of the assembled structure. The underfill encapsulant is subsequently cured to reach its optimized final properties. Currently, most encapsulated flip-chip packages are produced through this process.

A more efficient procedure is that used with a so-called no-flow fluxing underfill. In this process, the fluxing material is contained in the underfill, which is applied to the substrate prior to placement of the semiconductor component. After the component is placed, the full assembly is passed through a reflow oven, during which time the solder joints reflow and the underfill cures. The fluxing agent remains part of the cured underfill. In this process, the separate steps of applying the flux and post-curing the underfill are eliminated.

Typically, the underfill encapsulant is dispensed by syringe, which means that the viscosity must be sufficiently low for ease of dispensability. In the no-flow fluxing underfill operation, soldering and curing the underfill occur during the time in the reflow oven, which means that the underfill must maintain its low viscosity during the melting of the solder and cure rapidly after that. Currently in the industry, there is a demand for environmentally benign lead-free solders, such as, Sn/Ag with a melting point of 225°C and Sn/Ag/Cu with a melting point of 217°C. When these materials are used, the no-flow fluxing underfills must cure at higher temperatures.

US 2004/087681 A1 is directed to an underfill encapsulant composition especially useful in the no-flow underfill encapsulation process. The no-flow underfill compositions of said prior art comprise one or more epoxy resins and fluxing agents. US-B1-6 207 786 shows that the use of benzoxazines in combination with epoxy resins is useful in the electronic industry, especially as an underfill composition. WO 03/072638 A reveals the advantages of benzoxazines in the semiconductor fabrication industry and mentions that they can be used together with other curable resins which may include epoxy functionality. WO 01/34581 A discloses that the benzoxazines exhibit several advantages especially for forming composite parts, since they exhibit no out-gassing.

This invention is a composition for use as a fluxing underfill that contains a benzoxazine resin, an epoxy, and a fluxing agent, wherein the benzoxazine resin has the structure 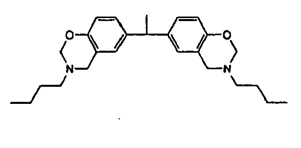 or
the structure

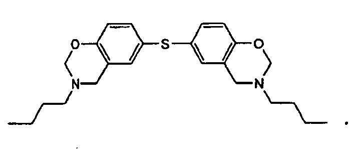

The following examples will disclose the synthesis of difunctional benzoxazine resins that are liquids at room temperature and that are suitable for use in no flow fluxing underfill compositions, compositions containing benzoxazines, and performance results. Suitable fluxing agents for these compositions include 1-naphthanoic acid, 1-naphthylacetic acid, and polysebacic polyanhydride (PSPA from Lonza). Suitable epoxy resins are commercially available and can be chosen as desired by the practitioner.

EXAMPLE 1: Synthesis

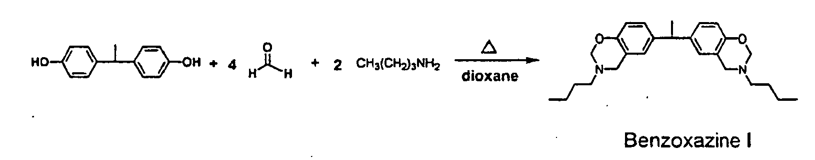

A two liter four-necked round-bottom flask equipped with an overhead mixer, condenser, addition funnel, and thermometer, was charged with 162.30 g of aqueous formaldehyde solution (37 wt% solution in water, 2.0 mol) and 400 mL of dioxane. The mixture was cooled by ice bath and the temperature was kept below 10°C. To this mixture 73.15 g of *n*-butylamine (1.0 mol) in 100 mL of dioxane was added dropwise. Upon completion of addition, the mixture was stirred for an additional 30 minutes. To this mixture was added 107.15 g of bisphenol E (0.5 mol) in 500 mL of dioxane. The temperature was then raised to the reflux temperature and the reaction was run overnight. After removal of solvent *in vacuo*, the viscous oil was dissolved in 800 mL of methyl-t-butyl ether (MTBE). The ether solution was washed with 3N aqueous sodium hydroxide solution (3 x 800 mL), followed by saturated sodium bicarbonate solution (3 x 600 mL), de-ionized water (3 x 800 mL), and saturated brine solution (400 mL). The organic layer was dried first over sodium sulfate and then silica gel. After removal of solvent *in vacuo*, 171.28 g of a yellowish liquid resin was obtained in a yield of 84%. The viscosity is 77,900 mPa.s at ambient temperature. ¹H NMR (CDCl₃, 400 MHz): δ 6.94 (d, 2H), 6.76 (s, 2H), 6.68 (d, 2H), 4.81 (s, 4H), 3.93 (s, 5H), 2.72 (t, 4H), 1.52-1.55 (m, 7H), 1.32-1.38 (m, 4H), 0.92 (t, 6H).

Example 2: Synthesis

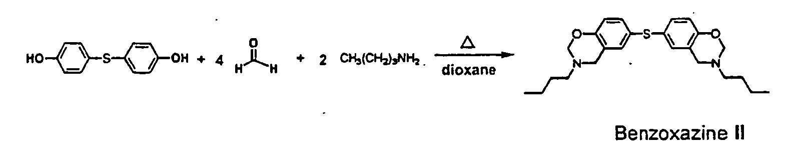

A 500mL three-necked round-bottom flask equipped with an overhead mixer, condenser, addition funnel, and thermometer, was charged with 32.46 g of aqueous formaldehyde solution (37 wt% solution in water, 0.40 mol) and 80 mL of dioxane. The mixture was cooled by ice bath and the temperature was kept below 10°C. To this mixture 14.63 g of n-butyl-amine (0.20 mol) in 20 mL of dioxane was added dropwise. Upon completion of addition, the mixture was stirred for an additional 30 minutes. To this mixture was added 21.83 g of 4,4'-thiodiphenol (0.10 mol) in 100 mL of dioxane. The temperature was then raised to the reflux temperature and the reaction was run overnight. After removal of solvent *in vacuo*, the viscous oil was dissolved in 200 mL of MTBE. The ether solution was washed with 3N aqueous sodium hydroxide solution (3 x 200 mL), followed by saturated sodium bicarbonate solution (200 mL), de-ionized water (2 x 200 mL), and saturated brine solution (200 mL). The organic layer was dried first over sodium sulfate and then over silica gel. After removal of solvent *in vacuo*, 22.46 g of a brownish liquid resin was obtained in a yield of 54%. The viscosity is 45,300 mPa.s at ambient temperature. ¹H NMR (CDCl₃, 400 MHz): δ 7.11 (d, 2H), 6.99 (s, 2H), 6.72 (d, 2H), 4.87 (s, 4H), 3.95 (s, 4H), 2.74 (t, 4H), 1.51-1.57 (m, 4H), 1.34-1.40 (m, 4H), 0.94 (t, 6H).

EXAMPLE 3: Forrnulations. Four compositions (A through D) were prepared to contain a benzoxazine from Example 1 or 2, a monobenzoxazine (benzoxazine III or IV) as a diluent, an epoxy resin, and the anhydride PSPA as a fluxing agent.

The monobenzoxazines used had the following structures:

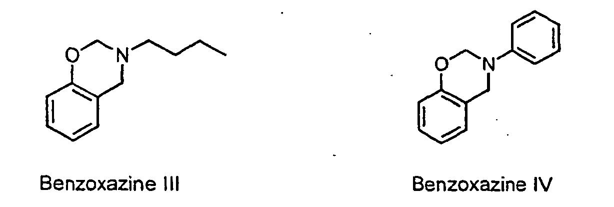

The epoxy resins used were Epiclon EXA850CRP from Dainippon Ink & Chemicals and XU71790-04L from Dow Chemical.

The fluxing agents used were 1-naphthanoic acid, 1-naphthylacetic acid, and polysebacic polyanhydride (PSPA from Lonza).

The components of the compositions were blended and passed through a three-roll mill three times at ambient temperature. The composition components are reported in parts by weight in Table 1.

| **Table 1: Composition Components** | **Ex 3 A** | **Ex 3 B** | **Ex 3 C** | **Ex 3 D** |
|---|---|---|---|---|
| Benzoxazine I | 30 | 30 | 30 | -**-** |
| Benzoxazine II | -- | -- | -- | 30 |
| Monobenzoxazine III | 10 | 10 | -- | -- |
| Monobenzoxazine IV | - | -- | 10 | 10 |
| Epoxy EXA-850CRP | 30 | 30 | 40 | 40 |
| Epoxy XU71790-04L | 10 | 10 | -- | -- |
| PSPA | 5 | 10 | 8 | 8 |

EXAMPLE 4. Performance. The benzoxazine compositions A through D from Example 3 were thermally cured by differential scanning calorimetry in which the cure onset temperature, cure peak temperature, and cure exotherm, were characterized using a DSC instrument (from TA Instruments, New Castle, Delaware). The glass transition temperatures (Tg) and coefficients of thermal expansion (CTE1, before Tg, and CTE2, after Tg) of the compositions were measured using a Thermal Mechanical Analyzer (model TMA 2920 from TA Instruments, New Castle, Delaware) on samples cured for two hours at 175°C.

To determine if the compositions were capable of fluxing solder, 0.2 grams of the composition were dispensed on a copper coupon, lead-free solder balls (Sn/Ag/Cu melting temperature 217°C) were dropped into the composition, a glass cover slide was placed over the composition, the assembly placed on a hot-plate pre-heated to 145°C for two minutes, then immediately transferred to another hot-plate pre-heated to 230-235°C for two minutes. This two-step reflow closely simulates the standard NIST reflow profile for lead-free solder. Fluxing results were evaluated by visual examination of the lead-free solders on copper coupons: flowing and spreading of the solder within the benzoxazine composition indicated fluxing occurred.

The performance results are reported in Table 2.

| Table 2 | **Ex 3 A** | **Ex 3 B** | **Ex 3 C** | **Ex 3 D** |
|---|---|---|---|---|
| DSC onset temp (°C)¹ | 211 | 208 | 213 | 207 |
| DSC peak temp (°C)¹ | 228 | 225 | 230 | 224 |
| DSC delta H (J/g)¹ | -232 | -139 | -227 | -187 |
| Lead-free solder flux on Cu coupon | Yes | Yes | Yes | Yes |
| Tg (°C) ² | 81.8 | 58 | 74.7 | 72 |
| CTE1 (ppm) ² | 65 | 72.8 | 67.7 | 68.4 |
| CTE2 (ppm) ² | 185 | 177 | 178 | 178 |
| Viscosity, mPa.s. 5rpm Spindle CP51 | 7,800 | 20,900 | 20,200 | 14,000 |

EXAMPLE 5: COMPARATIVE FORMULATIONS

Comparison compositions were prepared to contain benzoxazine, epoxy, and maleimide resins, without the inclusion of any fluxing agent. The benzoxazine was obtained from Vantico as product 97-191 and has the structure 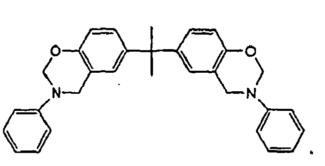 The epoxide used was a bis-A epoxide from Epiclon, product EXA850CRP. The phenolic resin used was HRJ1166 from Schenectady International. The maleimide used has the structure 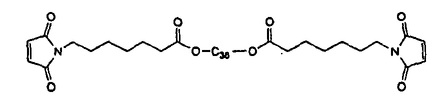 in which C₃₆ represents a linear or branched hydrocarbon chain that may contain a cyclic moiety.
The composition components, given in parts by weight, and fluxing performance results are reported in Table 3

| Table 3. Composition Components | Comparative Example 1 | Comparative Example 2 | Comparative Example 3 |
|---|---|---|---|
| Benzoxazine Resin | 10 | 35 | 25 |
| Epoxy Resin Epiclon | 10 | 70 | |
| EXA-850CRP | | | |
| Phenolic Resin | | 35 | |
| Maleimide Resin | | | 25 |
| Lead-free solder flux on | No | No | No |
| Cu coupon | | | |

In all the compositions in Table 1, the solders collapsed easily and spread on the substrate during fluxing tests. This indicated that the resin viscosity was low enough and the curing was delayed to enable the solder spreading at typical lead-free solder interconnection temperatures. When lead-free die was attached to the OSP substrate using the aforementioned encapsulant composition containing benzoxazine, good adhesion was also observed. The glass transition temperatures of the samples, which were cured for two hours at 175°C, were in the range of 58°C to 81.8°C without fillers. According to TMA results, the CTE1 and CTE2 were approximately 65-72.8 ppm and 177-185 ppm, respectively, without the presence of any fillers. In contrast, the compositions in Table 3 did not flux.

When a cure profile suitable for tin-lead solder (Tm = 183 °C) is desirable, a cationic catalyst can be employed in the aforementioned formulations to afford a cure peak at the desired temperature. Determination of the catalyst and amount needed can be determined without undue experimentation by the practitioner. Suitable cationic catalysts include, but are not limited to, carboxylic acid, HClO₄, BF₃, AlCl₃, AlBr₃, TiCl₄, l₂, SnCl₄, WCl₆, AlEt₂Cl, PF₅, VCl₄, AlEtCl₂, and BF₃ Et₂O. Preferred initiators include PCl₅, PCl₃, POCl₃, TiCl₄, SbCl₅, (C₆H₅)₃C⁺(SbCl₆)⁻, metallophorphyrin compounds such as aluminum phthalocyanine chloride.

## Claims

1. An underfill composition comprising
a benzoxazine resin,
an epoxy, and
a fluxing agent,
wherein the benzoxazine resin has
the structure 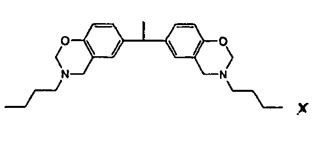 or
the structure

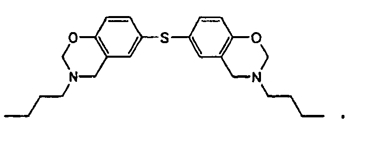

2. The underfill composition of claim 1 in which the fluxing agent is 1-naphthanoic acid, 1-naphthylacetic acid, or polysebacic polyanhydride.

## Patentansprüche

1. Underfill-Zusammensetzung, umfassend
ein Benzoxazin-Harz,
ein Epoxid und
ein Fluxmittel,
wobei das Benzoxazin-Harz die Struktur 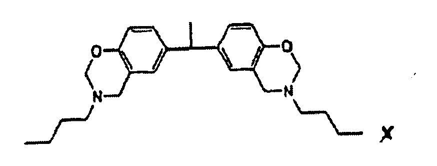 oder die Struktur 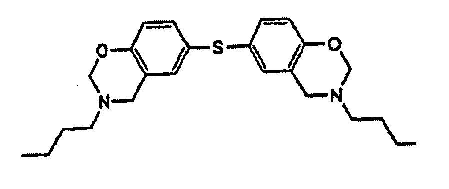 hat.

2. Underfill-Zusammensetzung nach Anspruch 1, in der das Fluxmittel 1-Naphthanosäure, 1-Naphthylessigsäure oder Polysebacinpolyanhydrid ist.

## Revendications

1. Composition de remplissage sous-jacent, comprenant :
une résine de benzoxazine,
un époxy, et
un agent fluxant,
dans laquelle la résine de benzoxazine a
la structure 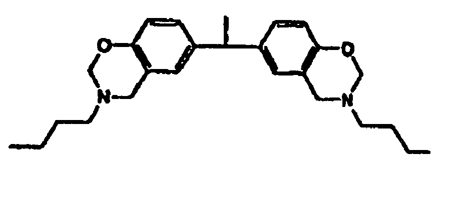 ou
la structure

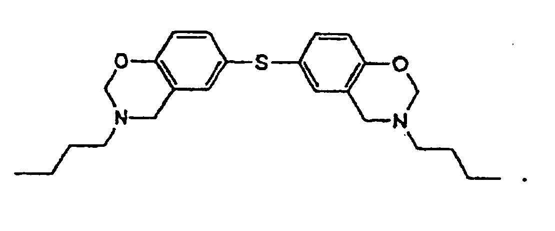

2. Composition de remplissage sous-jacent suivant la revendication 1, dans laquelle l'agent fluxant est l'acide 1-naphtanoïque, l'acide 1-naphtylacétique ou le polyanhydride polysébacique.
